# EUROPEAN PATENT APPLICATION

(11) **EP 3 342 493 A1**
(43) Date of publication of application: **04.07.2018**
(21) Application number: 15902314.2
(22) Date of filing: 06.11.2015
(51) Int. Cl.: B09B 3/00, B29B 17/02, C08J 11/06

(54) **WASTE MATERIAL TREATMENT APPARATUS AND WASTE MATERIAL TREATMENT METHOD**

(30) Priority: 24.08.2015 JP 2015165210
(71) Applicant: Kentech Institute Corporation, Tokyo 110-0005 (JP)
(72) Inventor: YOKOI, Shoji, Tokyo 110-0005 (JP); HANG, Pengzhi, Tokyo 110-0005 (JP); ASANO, Yukinori, Tokyo 110-0005 (JP)
(74) Representative: Erhardt, Martin
(86) International application number: PCT/JP2015/081346
(87) International publication number: WO 2017/033345

(57) **Abstract**

[Problem]

To provide a waste material treatment apparatus that renders waste materials harmless and reduces the volume thereof while restraining generation of dioxins as much as possible, and that at the same time enables recycling of plastics. [Solution] In order to solve the above problems, provided is a waste material treatment apparatus which: supplies a reactant to facilitate a prompt reaction between an oxidation agent or a neutralizing agent and gases that contain chlorine, hydrocarbons, etc., and that are emitted from non-plastic garbage present in waste materials when heated instantaneously; renders the non-plastic garbage harmless and reduced in volume without generating dioxins or carbon dioxide by repeating the instantaneous heating; and renders plastics harmless and cleaned, thereby enabling recycling thereof.

## Description

### Technical Field

The present invention relates to a waste material treatment apparatus in which waste materials are rendered harmless and a volume thereof is reduced without generating dioxin being a hazardous substance, carbon dioxide being a causative substance of global warming, or the like, and also plastics contained in the waste materials can be recycled.

### Background Art

Waste materials from a general household contain miscellaneous refuse such as pieces of paper, food residues and plastics, and desirably suitably treated according to types of refuse. For example, volume reduction or suppression of generation of malodor is preferable for the pieces of paper or the food residues, or recycling is preferable for the plastics or the like. Moreover, medical waste materials containing, as a mixture, blood or pieces of tissue derived from medical practice and disposed of, and medical instruments, containers, gauze or the like having such material attached thereon may be a contamination source of causing an infectious disease or the like, and therefore such materials should be rendered harmless and suitably treated. Moreover, recycling is also preferable for the plastics contained in the medical waste materials in a manner similar to the case of general waste materials. In order to reduce the volume of the waste materials described above or render the waste materials harmless, incineration is simple and effective. However, when the waste materials containing, as the mixture, the plastics are incinerated without discretion, a hazardous substance such as dioxins is liable to be discharged based on chlorine produced by combustion of the plastics.

As an art for treating the waste materials containing, as the mixture, the plastics, for example, Patent Literature 1 discloses an art in which waste materials containing plastics are indirectly heated at 450°C to 650°C and converted into oil, and gases produced by thermal decomposition are heated at 900°C to 1200°C, and cracked. According to the art described above, recycling is achieved by converting the plastics into oil, and also the gases such as dioxins produced by the thermal decomposition are decomposed by heating at high temperature, and rendered harmless.

### Citation List

### Patent Literature

Patent Literature 1: JP 2000-202419 A

### Summary of Invention

### Technical Problem

The art disclosed in Patent Literature 1 requires facilities such as a furnace which can withstand heating at high temperature, and a large amount of energy for heating waste materials at high temperature to have a problem of being obliged to bear a significant treatment cost. Accordingly, an object of the present inventors is to provide a waste material treatment apparatus in which conditions of generating dioxins to cause the significant treatment cost are eliminated to render the waste materials harmless and reduces a volume thereof while also suppressing generation of carbon dioxide as much as possible, and also plastics or the like can be recycled.

### Solution to Problem

Therefore, in order to solve the problem described above, the present invention provides a waste material treatment apparatus or the like as described below. More specifically, the present invention provides a waste material treatment apparatus including: a chamber for heating treatment of waste materials; a crushed piece supply port for supplying crushed pieces of waste materials to the chamber; a crushed piece instantaneous temperature increase body heated by a heater for repeatedly performing instantaneous temperature increase of the crushed pieces supplied into the chamber; a heater control unit capable of controlling the heater, when plastics are contained in the crushed pieces, in such a manner that a temperature of the plastics is instantaneously increased to a temperature in the neighborhood of a softening temperature of the plastics by the crushed piece instantaneous temperature increase body; a reactant supply unit including a reactant supply hole for supplying a reactant being an oxidation agent and a neutralizing agent arranged in the neighborhood of the crushed piece instantaneous temperature increase body to allow gases produced from the crushed pieces by temperature increase to react therewith in the neighborhood of the crushed piece instantaneous temperature increase body; an air mixture gas supply unit for supplying, to the chamber, an air mixture gas capable of cooling, instantaneously when instantaneously temperature-increased crushed pieces by coming close to the crushed piece instantaneous temperature increase body are isolated from the crushed piece instantaneous temperature increase body, the crushed pieces isolated therefrom; and an exhaust unit having a negative pressure outlet for negatively pressurizing an inside of the chamber to discharge gases inside the chamber to an outside.

Moreover, the present invention provides the waste material treatment apparatus including the configuration described above, wherein the gas supply unit has an ozone mixing means for mixing ozone in air. Moreover, the present invention provides the waste material treatment apparatus including any one of the configurations described above, wherein the negative pressure outlet is provided in an upper portion of the chamber. Moreover, the present invention provides the waste material treatment apparatus including any one of the configurations described above, further including an intra-chamber gas analysis unit, wherein the heater control unit has an intra-chamber gas-dependent control means for controlling the heater in corresponding to analysis results of intra-chamber gases in the intra-chamber gas analysis unit. Moreover, the present invention provides the waste material treatment apparatus including any one of the configurations described above, wherein the reactant supply hole is provided for the crushed piece instantaneous temperature increase body.

Moreover, the present invention provides the waste material treatment apparatus including any one of the configurations described above, wherein the crushed piece instantaneous temperature increase body is a needle body which performs an insertion and retraction movement relative to the crushed pieces inside the chamber. Moreover, the present invention provides the waste material treatment apparatus including any one of the configurations described above, having a crushed piece agitation mechanism for agitating the crushed pieces inside the chamber. Moreover, the present invention provides the waste material treatment apparatus including any one of the configurations described above, wherein the crushed piece instantaneous temperature increase body is a fixed block arranged in such a manner that the crushed pieces agitated inside the chamber instantaneously come close thereto.

Moreover, the present invention provides a method for treating waste materials, including: a crushing step of crushing waste materials into small pieces; an instantaneous temperature increase step of instantaneously increasing, when plastics are contained in crushed pieces crushed in the crushing step, a temperature of the plastics to about a temperature of a softening temperature thereof; a decomposition step of releasing urea, instantaneously when the temperature is instantaneously increased in the instantaneous temperature increase step, to a place near temperature-increased crushed pieces to decompose the urea into an ammonia gas and an intermediate of cyanic acid at a temperature near the crushed pieces; a reaction step of allowing both substances decomposed in the decomposition step to react with various gases generated from instantaneously temperature-increased crushed pieces; and a cooling step of cooling substances produced by the reaction in the reaction step. Moreover, the present invention provides the method for treating the waste materials, including each step described above, wherein the reaction step further has a hydroxide reaction substep of releasing hydroxide to a place near the crushed pieces to allow reaction therewith.

Moreover, the present invention provides a method for treating crushed pieces being waste materials, including: an instantaneous temperature increase step of instantaneously increasing a temperature in the neighborhood of a surface of the crushed pieces to a temperature in the neighborhood of a softening point thereof; a temperature decrease step of instantaneously decreasing the temperature in the neighborhood of the surface of instantaneously temperature-increased crushed pieces; and a neutralizing gas supply step of supplying a neutralizing gas to a place in the neighborhood of the surface of the instantaneously temperature-increased crushed pieces.

Moreover, the present invention provides a method for treating crushed pieces being waste materials, including: an instantaneous temperature increase step of instantaneously increasing a temperature in the neighborhood of a surface of the crushed pieces to a temperature in the neighborhood of a softening point thereof; and a neutralizing gas supply step of supplying a neutralizing gas having a temperature relatively lower than the temperature in the neighborhood of the softening point thereof to a place in the neighborhood of the surface of instantaneously temperature-increased crushed pieces to instantaneously decrease the temperature in the neighborhood of the surface of the instantaneously temperature-increased crushed pieces.

Moreover, the present invention provides a method for treating crushed pieces being waste materials, including: an instantaneous temperature increase step of instantaneously increase a temperature in the neighborhood of a surface of the crushed pieces to a temperature in the neighborhood of a softening point thereof; a neutralizing gas supply step of supplying a neutralizing gas to a place in the neighborhood of the surface of instantaneously temperature-increased crushed pieces to neutralize an active gas generated from the place in the neighborhood of the surface of the crushed pieces; and a temperature decrease step of instantaneously decreasing the temperature in the neighborhood of the surface of the instantaneously temperature-increased crushed pieces .

Moreover, the present invention provides a method for treating crushed pieces being waste materials, including: an instantaneous temperature increase step of instantaneously increasing a temperature in the neighborhood of a surface of the crushed pieces to a temperature in the neighborhood of a softening point thereof; and a neutralizing gas supply step of supplying a neutralizing gas having a temperature relatively lower than the temperature in the neighborhood of the softening point thereof to a place in the neighborhood of the surface of instantaneously temperature-increased crushed pieces to neutralize an active gas generated from the place in the neighborhood of the surface of the crushed pieces, and also instantaneously decrease the temperature in the neighborhood of the surface of the instantaneously temperature-increased crushed pieces.

### Advantageous Effects of Invention

The present invention can provide a waste material treatment apparatus in which organic waste materials are rendered harmless and a volume thereof is reduced, and plastics can be recycled while suppressing discharge of a hazardous substance such as dioxins as much as possible.

### Brief Description of Drawings

Figure 1 is a conceptual diagram showing phases in corresponding to temperatures of plastics and non-plastic refuse contained in waste materials.
Figure 2 is a conceptual diagram showing one example of a waste material treatment apparatus according to Embodiment 1.
Figure 3 is a conceptual diagram showing one example of the waste material treatment apparatus according to Embodiment 1.
Figure 4 is a conceptual diagram for explaining action of crushed pieces as produced by contact with a crushed piece instantaneous temperature increase body.
Figure 5 is a conceptual diagram showing one example of a reactant supply hole and a crushed piece instantaneous temperature increase body.
Figure 6 is a flowchart showing one example of a treatment process of the waste material treatment apparatus according to Embodiment 1.
Figure 7 is a conceptual diagram showing another example of the waste material treatment apparatus according to Embodiment 1.
Figure 8 is a conceptual diagram showing one example of a waste material treatment apparatus according to Embodiment 3.
Figure 9 is a conceptual diagram showing one example of the waste material treatment apparatus according to Embodiment 3.
Figure 10 is a conceptual diagram showing one example of a needle body for which a reactant supply hole is provided.

### Description of Embodiments

Hereinafter, Embodiments according to the present invention will be described with reference to drawings. In addition, the present invention is not limited by the Embodiments at all, and may be practiced in various aspects within the scope without departing from the spirit.

Embodiment 1 mainly relates to claims 1 to 3, 7 to 14 or the like. Embodiment 2 mainly relates to claim 4 or the like. Embodiment 3 mainly relates to claims 5, 6 or the like.

### <Embodiment 1>

### <Embodiment outline>

Waste materials mainly assumed as objects to be treated by a waste material treatment apparatus according to the present embodiment are waste materials containing, as a mixture, pieces of paper, food residues, fat and oil, excrement, blood, pieces of tissue, gauze or the like, and containers in which such items are stored, instruments with which such items are brought into contact, or the like. Plastics are used for the containers or the instruments in many cases, and therefore the waste materials mainly assumed as the objects to be treated are materials containing, as a mixture, the plastics and non-plastic refuse being refuse other than the plastics.

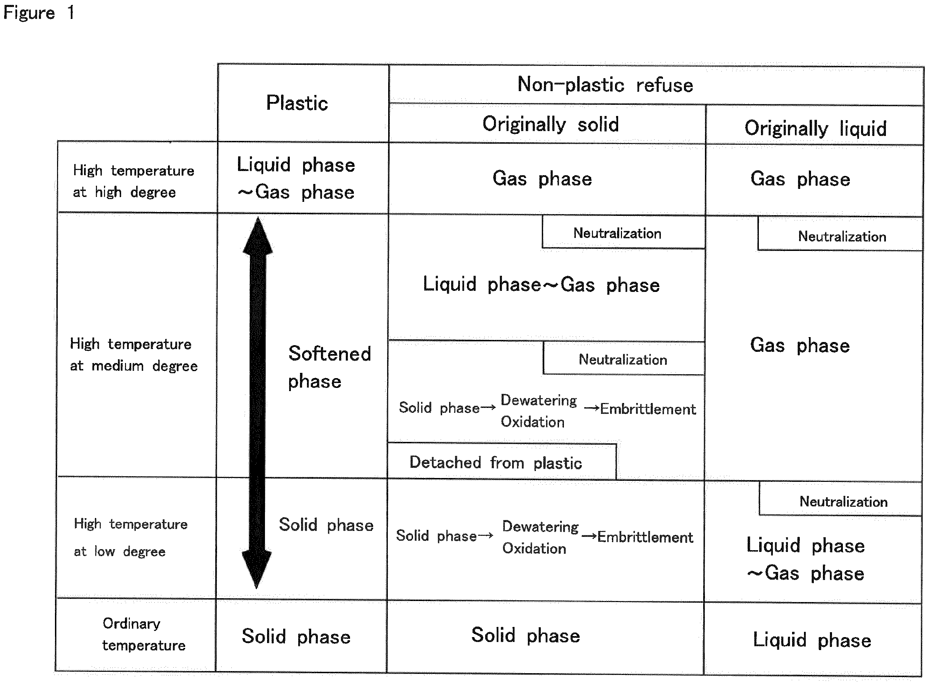

Figure 1 is a conceptual diagram showing phases in corresponding to temperatures of plastics and non-plastic refuse contained in waste materials. As shown in the figure, the plastics are softened in a state of relatively high temperature at a medium degree. In addition, a softened phase is not a technical term, but for convenience of explanation, the softened phase means a state in which softening is caused, although not formed into a liquid phase. In the present apparatus, heat at a degree at which the plastics is instantaneously formed into the softened phase is applied as an upper limit. Such a heating range is shown by a double-headed arrow in the figure. The upper limit of heating as described above is determined. Thus, neither chlorine, hydrocarbon or the like is generated from the plastics by thermal decomposition, nor a reaction of forming of dioxins, carbon dioxide or the like is caused.

Action of heating in such a range on the non-plastic refuse will be described. First, in the non-plastic refuse, with regard to materials in a solid at ordinary temperature, such as pieces of paper, pieces of tissue and rubber, surfaces of the crushed pieces are continuously oxidized, and dried into fragile matter by a strong oxidizing gas (such as ozone) under an atmosphere from ordinary temperature to around 100°C filled inside the chamber, and also such refuse is pulverized by explosive evaporation or rapid evaporation caused by overheating of moisture thereinside, and micronized to reduce the volume thereof as a whole. Further, tissue of the refuse is embrittled by dewatering action into decomposition or disintegration of the tissue. With regard to the non-plastic refuse attached on the surfaces of the plastics, the non-plastic refuse is detached from the surfaces of the plastics by such embrittlement or disintegration caused by the dewatering action. Moreover, when fungi or microorganisms are contained in the non-plastic refuse, most of the fungi or the microorganisms are killed by heating. Thus, the volume of the non-plastic refuse is reduced and the non-plastic refuse is rendered harmless by dewatering or embrittlement of the tissue by heating in the range described above, and the plastics having the non-plastic refuse attached thereon are cleaned and rendered harmless.

Moreover, the refuse is liquefied or vaporized or gases are produced depending on kinds of refuse in several cases. Moreover, chemical decomposition is also caused by heating, and a decomposed gas is generated in several cases. Moreover, with regard to the non-plastics in which most of the compositions is occupied by a liquid at ordinary temperature, such as blood, food residues in a liquid state, chemicals and excrement, contained moisture composing the non-plastics is evaporated or vaporized by heating. Also in the processes, gases are produced by the decomposed gas or vaporization by heating. Specific examples of the gasses include hydrogen, oxygen, chlorine, nitrogen, hydrocarbon and carbon oxide. The gases are produced in an active state, and therefore the generated gases react with each other to form dioxins, carbon dioxide or the like in several cases.

Therefore, in the present waste material treatment apparatus, a reactant being an oxidation agent and a neutralizing agent is supplied to a place near a heating place in which the above gases may be generated, and the gasses generated are allowed to react with the reactant to form a stable compound, thereby inhibiting a reaction to dioxins or the like. Further, an air mixture gas for cooling is supplied to the place near the heating place in which the gasses may be generated, thereby immediately terminating the reaction to dioxins.

As described above, in the present waste material treatment apparatus, the non-plastic refuse is rendered harmless and a volume thereof is reduced without forming dioxins or carbon dioxide by repeating instantaneous heating, while immediately neutralizing, with the oxidation agent, the gases containing chlorine, hydrocarbon or the like generated from the non-plastic refuse, and also the plastics are cleaned and rendered harmless. Thus, recycling can be made.

### <Embodiment 1 configuration>

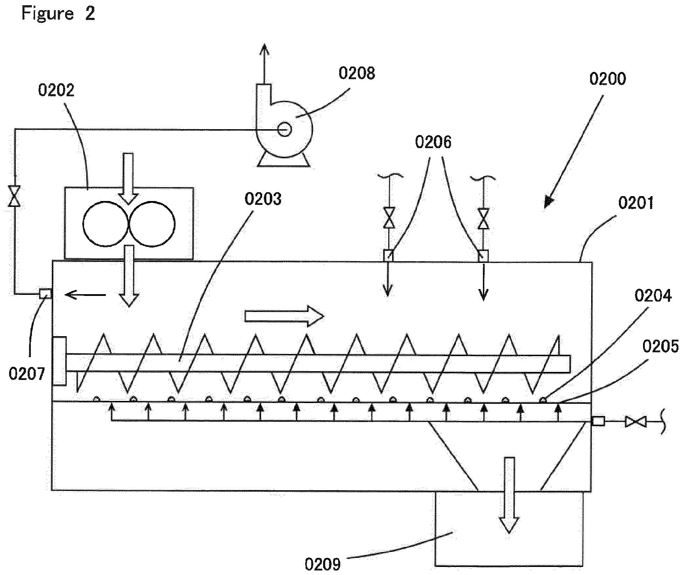

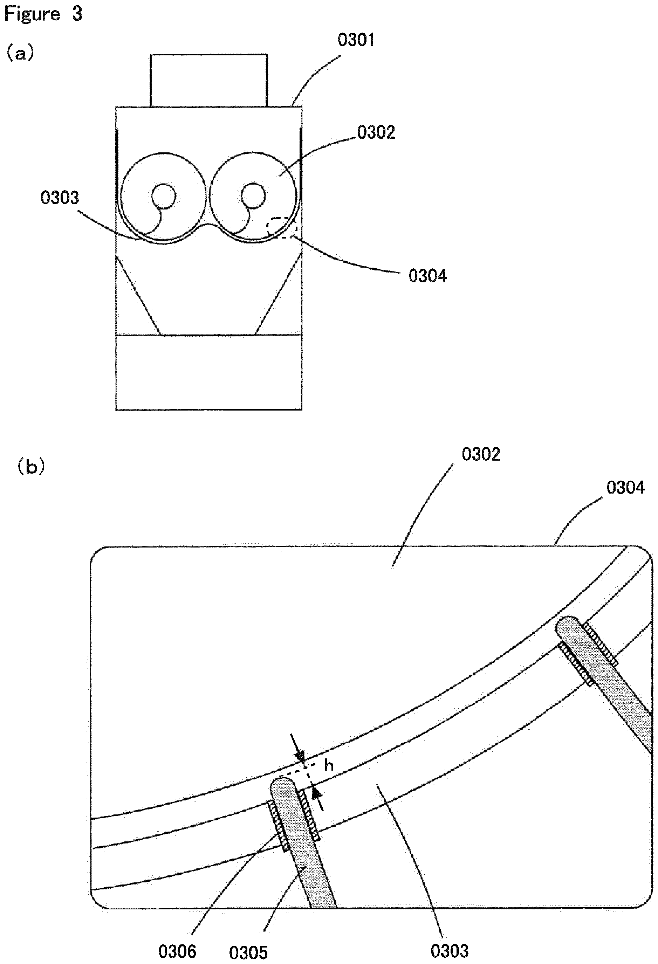

A main configuration of the waste material treatment apparatus according to the present embodiment has a "chamber," a "crushed piece supply port," a "crushed piece instantaneous temperature increase body," a "heater control unit," a "reactant supply unit," an "air mixture gas supply unit," and an "exhaust unit." Each configuration will be described below with reference to Figure 2 and Figure 3. Figure 2 is a conceptual diagram showing one example of the waste material treatment apparatus according to Embodiment 1. Figure 3 is a cross-sectional view of the present apparatus shown in Figure 2 when viewed from a conveyance direction of the crushed pieces, and an enlarged view of a part thereof.

A "waste material treatment apparatus" (0200) according to the present embodiment has a "chamber" (0201) for applying heating treatment to the waste materials. The chamber is not particularly limited as long as the chamber has a material and a structure which can withstand the heat treatment, and may be a chamber according to a publicly-known technology. Moreover, an atmosphere inside the chamber is preferably mainly an inert gas such as nitrogen. The reason is that the present apparatus causes heating of the waste materials as described later, but is not intended for combustion. Moreover, the chamber has airtightness during operation in order to negatively pressurize an inside of the chamber as described later.

The "exhaust unit" has a "negative pressure outlet" (0207) for negatively pressurizing the inside of the chamber to discharge gasses inside the chamber to an outside, in which the inside of the chamber is negatively pressurized by using a pump (0208) or the like. As described later, in the present apparatus, the reactant and the air mixture gas are supplied to a place in the neighborhood of the crushed piece instantaneous temperature increase body or the like. The reactant and the air mixture gas to be supplied therein is provided with flowability toward an outlet by converting the inside of the chamber in a negatively pressurized state. Resultant action will be described in detail in a description on the reactant supply unit or the air mixture gas supply unit.

Upon discharging the gasses inside the chamber to the outside from the exhaust unit, the gases are also preferably passed through a filter or a catalyst and then discharged in suppressing air pollution. Moreover, the gasses discharged from the inside of the chamber may be conveyed to another gas purifying apparatus or the like.

A "crushed piece supply port" (0202) is provided for supplying the crushed pieces of the waste materials to the chamber. In Figure 2, the port provided with a crusher for crushing the waste materials is shown. When the waste materials already crushed by a crusher different from the present apparatus are supplied, the crushed piece supply port has no necessity of providing such a crusher. In addition, the crushed pieces crushed into a size as small as about 5 to about 10 mm are preferable in efficiently treating the crushed pieces. Moreover, the crushed piece supply port is configured in such a manner that the airtightness is kept during non-supply of the crushed pieces.

Moreover, with regard to supply of the crushed pieces, the crushed pieces may be supplied therein by free fall from the crushed piece supply port, or may be supplied therein while the crushed pieces are being pressed in a direction in which the crushed pieces are to be conveyed by using an actuator or the like. Treatment efficiency at which the crushed pieces are treated in a dense state thereof can be improved by squeezing and supplying the crushed pieces thereinto. Moreover, conveyance of the crushed pieces can be further reinforced by squeezing the crushed pieces thereinto, and a treatment speed and the treatment efficiency can be improved.

The waste materials mainly assumed as objects to be treated by the present apparatus are waste materials containing, as a mixture, pieces of paper, food residues, fat and oil, excrement, blood, pieces of tissue, gauze or the like, and containers in which such items are stored, or instruments with which such items are brought into contact. More specifically, the objects are the waste materials containing, as a mixture, plastics which are used in the containers or the instruments in many cases, and the plastic refuse a surface of which is contaminated with contents thereof or the like, and the non-plastic refuse being refuse other than the plastics, such as pieces of paper, food residues, fat and oil, excrement, blood, pieces of tissue, and gauze.

The chamber is equipped with a "screw conveyer" (0203) as a means for conveying the crushed pieces while being agitated, and the crushed pieces supplied from the crushed piece supply port are treated while being conveyed in a right direction toward the figure as shown by a void arrow in the figure.

A bottom surface of the chamber is equipped with a "crushed piece instantaneous temperature increase body" (0204) heated by a heater for repeatedly performing instantaneous temperature increase of the crushed pieces supplied therein. The crushed piece instantaneous temperature increase body will be described also with reference to Figure 3. As shown in Figure 3(a), a "chamber" (0301) is thereinside equipped with a "screw conveyer" (0302) for conveying the crushed pieces supplied therein while being agitated, and a "bottom surface" (0303) of the chamber is formed with being slightly isolated from an outermost periphery of a screw.

Figure 3 (b) is an enlarged view of a part (0304) in which the screw comes close to the bottom surface of the chamber. As shown in the figure, a hole is perforated in a bottom portion of the chamber, and a "crushed piece instantaneous temperature increase body" (0305) is arranged in a state in which the crushed piece instantaneous temperature increase body (0305) is slightly projected from the bottom surface toward the screw. Moreover, a "heat insulation material" (0306) is arranged in an edge of the hole to suppress transmission of heat of the crushed piece instantaneous temperature increase body to the bottom surface of the chamber.

The crushed piece instantaneous temperature increase body is heated by a heater (not shown) to 120°C to 300°C, for example, in corresponding to contents of the organic waste materials to be treated. In a special case, when residues having a large amount of inorganic substances containing a small amount of organic substances (for example, a content of the organic substances is 5% by weight or less) are treated as the objects to be treated, an instantaneous heating temperature may be over 300°C, and for example, may be 500°C to 600°C in several cases. In the above case, the present apparatus is used for the purpose of collecting general industrial waste materials, residues generated from a specific production process, impurities of salts or the like by taking advantage of features of a treatment process in the present apparatus. In the above case, a recycling method is available in which, when a large amount of inorganic substances containing salts is contained, a heating temperature is set to a somewhat high level so as to come close to a melting point of the inorganic substances such as salts thereof, or impure organic substances mixed in the inorganic salts and to be collected are heated to cause oozing of the impure organic substances to be volatilized, and the salts are purified and collected. When the waste materials contain a large amount of salts and the impure organic substances have a predetermined concentration or less, however, the above method can be applied, and in the above case, a measurement and a test or an examination is required in several cases. A plurality of such crushed piece instantaneous temperature increase bodies are arranged in the chamber, and for example, about 2 to about 10 units are arranged per 10 cm-square region. Moreover, in the example in Figure 2, an isolation distance between the bottom surface of the chamber and the outermost periphery of the screw is a little less than 10 mm, and the crushed piece instantaneous temperature increase body is configured to be projected from the bottom surface of the chamber at a height (h) of about 5 mm. Moreover, a length of the crushed piece instantaneous temperature increase body in the conveyance direction is about 10 to about 30 mm, for example.

The crushed pieces are conveyed while being agitated inside the chamber by the screw conveyer, and the plurality of crushed piece instantaneous temperature increase bodies each having a length of about 10 to about 30 mm in the conveyance direction are discretely arranged. Further, the bottom surface of the chamber in which no crushed piece instantaneous temperature increase body is arranged is adjusted to a temperature lower than the temperature of the crushed piece instantaneous temperature increase body by means of the heat insulation material. Therefore, the crushed pieces are exposed to heating at a relatively high temperature only instantaneously when the crushed pieces are brought into contact with the crushed piece instantaneous temperature increase body, and if the crushed pieces are isolated from the crushed piece instantaneous temperature increase body, the temperature is immediately decreased. Accordingly, the temperature increase of the crushed pieces by the crushed piece instantaneous temperature increase body is made in a significantly short period of time, and can be reasonably maintained to be an instantaneous temperature increase. Then, the crushed pieces are repeatedly brought into contact with the crushed piece temperature increase body and are isolated therefrom during being conveyed as described above. Thus, the instantaneous temperature increase is repeated. In addition, a conveyance speed in the screw conveyer is generally 0.1 cm/sec to 15 cm/sec, and is appropriately selected depending on a kind, an amount or the like of the crushed pieces.

Here, the heater for heating the crushed piece instantaneous temperature increase body is controlled by the heater control unit. The heater control unit causes control of the heater in such a manner that the crushed pieces composed of instantaneously temperature-increased plastics to a temperature in the neighborhood of a softening temperature thereof by the crushed piece instantaneous temperature increase body. The softening temperature means a temperature at which the plastics are softened by being heated, and an expression "a temperature is instantaneously increased to a temperature in the neighborhood of a softening temperature" means that the temperature of the crushed piece is increased to a degree at which only a limited surface of the crushed piece is softened. An expression "only a limited surface is softened" means that the surface is softened at about 5 to about 1000 µm in a depth direction from the surface of the crushed piece. In addition, a softening point of polyvinyl chloride is 170°C, a softening point of polyethylene is 95°C to 140°C, a softening point of polystyrene is approximately 100°C, and a softening point of polyethylene terephthalate is 255°C. The temperature of the crushed piece can be instantaneously increased to the temperature in the neighborhood of the softening point thereof by allowing the crushed piece to reach the softening temperature as described above for only a limited period of time.

In order to instantaneously increase the temperature of the crushed pieces composed of the plastics to the temperature in the neighborhood of the softening temperature thereof, the heater is controlled in such a manner that the temperature of the crushed piece instantaneous temperature increase body reaches a level higher than the temperature in the neighborhood of the softening temperature. The temperature is controlled to the temperature in corresponding to the configuration of the crushed piece instantaneous temperature increase body, the objects to be treated or the like, and for example, the heater is set in such a manner that the crushed piece instantaneous temperature increase body has a temperature higher by about 40°C to about 70°C than the softening temperature of the plastics. More specifically, for example, the heater is controlled to 210°C to 230°C in the case of polyvinyl chloride, 135°C to 160°C in the case of polyethylene, 140°C to 155°C in the case of polystyrene, and 295°C to 320°C in the case of polyethylene terephthalate, and so forth. In addition, when the plastics in the waste materials to be treated can be specified to one kind, the heater only needs be controlled in corresponding to the softening temperature of the plastic of the kind, but when a plurality of kinds of plastics are mixed, the heater is controlled in corresponding to the highest softening temperature thereof. In the above case, generation of gases from the plastics having a low softening temperature is concerned. In reality, however, when a temperature difference among the softening temperatures of the plastics is about 100°C to about 150°C, no gases are generated, and only a large amount of softened portions is produced in the plastics having the low softening temperature. More specifically, softening of the plastics having the high softening temperature only needs take precedence.

With regard to the action of the crushed piece instantaneous temperature increase body heated by the thus controlled heater on the crushed pieces, when the crushed pieces are pieces of paper, pieces of wood, fibers of gauze, or the like, moisture contained in the crushed pieces is heated and evaporated, and a remaining carbon compound or the like which composes the crushed pieces is decomposed and softened. Accordingly, the volume of the above crushed pieces can be reduced. Moreover, even if fungi and so forth penetrate into the crushed pieces or are attached onto the surface thereof, the fungi are killed by being heated. Accordingly, the volume of the non-plastic refuse is reduced and the non-plastic refuse is rendered harmless by means of the crushed piece instantaneous temperature increase body.

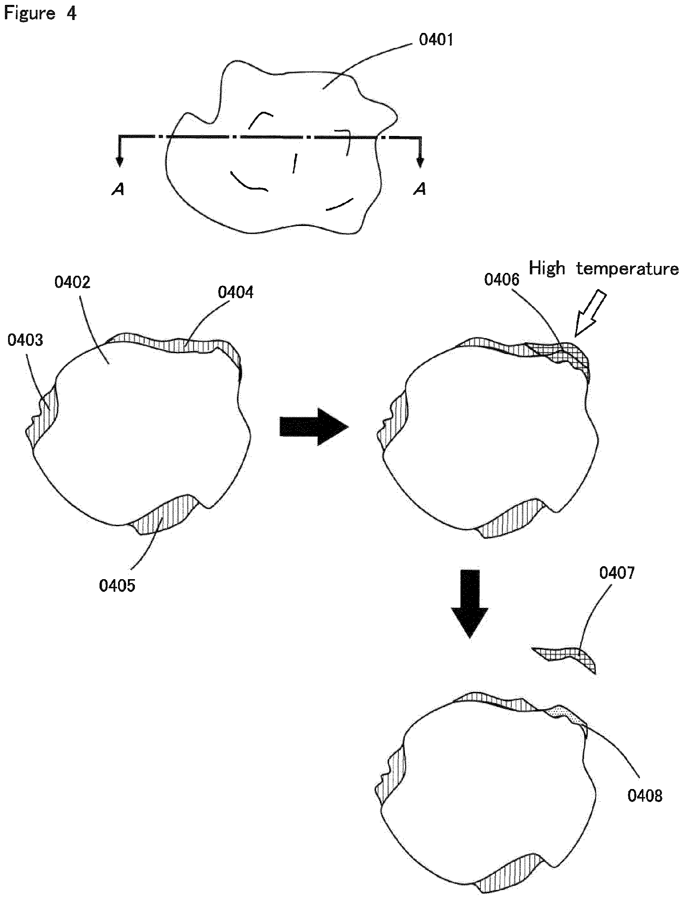

Next, a case where the crushed pieces are the plastic having the non-plastic refuse such as blood, pieces of tissue and food residues attached on the surface thereof will be described with reference to Figure 4. Figure 4 is a conceptual diagram for explaining action of the crushed pieces produced by contact with the crushed piece instantaneous temperature increase body. In the figure, a crushed piece (0401) being plastics having an attachment on a surface thereof, and a cross section (0402) along an A-A line of the crushed piece. As shown in the figure, attachments (0403, 0404, 0405) are attached on the surface of the plastics. If the crushed piece is brought into contact with the crushed piece instantaneous temperature increase body, a part (0406) in contact therewith is brought into a high temperature. Then, as described above, an attachment (0407) is brought into contact with the crushed piece instantaneous temperature increase body, thereby being dried to cause moisture evaporation and issue disintegration, resulting in reduction of adhesive force with the surface of the plastics. Moreover, a surface (0408) of temperature-increased plastics by contact with the crushed piece instantaneous temperature increase body, in which softening may be caused. Such possibility also may contribute to reduction of adhesive force with the attachment. The attachments are detached from the surface of the plastics by the action described above. With regard to the crushed pieces composed of the plastics having the attachments on the surface thereof as described above, the plastics are cleaned and rendered harmless, and recycling thereof can be made by separating the plastics from the organic compounds such as blood and pieces of tissue attached on the surface by contact with the crushed piece instantaneous temperature increase body.

In addition, the crushed piece is not necessarily in a lump shape as shown in Figure 4. For example, the crushed piece may be in a thin plate shape or a bent plate shape. In such a case, even if the crushed piece is brought into contact with the crushed piece instantaneous temperature increase body, the plastic refuse attached on the crushed piece is not directly brought into contact with the crushed piece instantaneous temperature increase body in several cases. However, the non-plastic refuse attached on the crushed piece is heated by temperature increase of the crushed piece by heating of the crushed piece instantaneous temperature increase body. Thus, dewatering, tissue disintegration or the like is caused, and the plastics which compose the crushed piece are cleaned and rendered harmless.

Here, a decomposition reaction is caused in the attachments on the plastics by being heated, and the gasses such as chlorine, hydrogen and hydrocarbon are generated in several cases. The gases further react therewith into dioxins or carbon dioxide. Therefore, in the waste material treatment apparatus, the reactant such as the oxidation agent and the neutralizing agent is supplied to the place near the heating place in which the gases may be generated to allow the gases generated to react with the reactant being the oxidation agent and the neutralizing agent to form a stable compound, thereby inhibiting formation of a dioxins precursor gas or the like into dioxins to inhibit generation of dioxins or the like.

To explain in a further generalized manner, when the non-plastic refuse contained in the crushed pieces is heated, chlorine, hydrogen, carbon monoxide, various hydrocarbons and the like are produced in several cases. The substances are alkaline or acidic, and all are produced in an active state. Accordingly, the substances are liable to cause various reactions with each other to form a hazardous substance such as dioxins, an unintended substance or an unknown substance. When the unintended substance or the unknown substance is formed, it becomes difficult to apply suitable treatment for rendering the substance harmless. Moreover, rendering of dioxins harmless is made at a high cost as already described.

Therefore, the oxidation agent and the neutralizing agent are immediately supplied to alkaline or acidic active substances produced by heating the non-plastic refuse contained in the crushed pieces to promote a reaction of forming an inactive and stable salt. Thus, unwanted various reactions described above are inhibited.

Therefore, the present apparatus has the reactant supply unit for supplying the reactant such as the oxidation agent and the neutralizing agent. The reactant supply unit includes a reactant supply hole arranged in the neighborhood of the crushed piece instantaneous temperature increase body to allow the gases produced from the crushed pieces by temperature increase to react therewith in the neighborhood of the crushed piece instantaneous temperature increase body. In Figure 2, a "reactant supply hole" (0205) is provided on the bottom surface of the chamber.

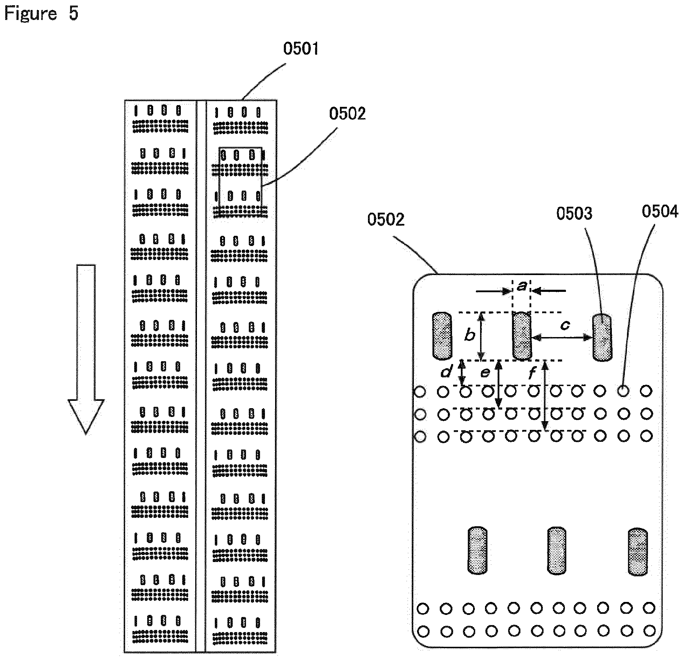

Figure 5 is a conceptual diagram showing one example of a reactant supply hole and a crushed piece instantaneous temperature increase body provided in a bottom portion of a chamber. A left figure schematically shows an exterior view of a "bottom portion of the chamber" (0501), and a void arrow shows a direction in which the crushed pieces are conveyed. Moreover, a right figure shows an enlarged view of one region (0502) thereof. As shown in the right figure, a plurality of "crushed piece instantaneous temperature increase bodies" (0503) are arranged in the bottom portion of the chamber, and a great number of "reactant supply holes" (0504) are provided near on a downstream side of the conveyance direction. The crushed piece instantaneous temperature increase body has, for example, a length a of about 10 mm in a lateral direction, and a length b of about 30 mm in a longitudinal direction, and has a height of about 5 mm from the bottom of the chamber. Moreover, an interval c of the crushed piece instantaneous temperature increase body in a lateral direction is about 40 mm. An inner diameter of the reactant supply hole is about 1 to about 5 mm. Moreover, a distance d from the most downstream end of the crushed piece instantaneous temperature increase body in the conveyance direction to the reactant supply hole in the nearest neighbor column thereof is about 15 mm, and a distance e from the most downstream end thereof to the reactant supply holes in the next neighbor column thereof is about 30 mm, and a distance f from the most downstream end thereof to the reactant supply hole in the remotest column thereof is about 45 mm.

In addition, values of the crushed piece instantaneous temperature increase body and an arrangement thereof are represented as one example, and can be appropriately determined in corresponding to a dimension and a capacity of the chamber, a kind and an amount of the waste materials to be treated, or the like. For example, the values can be appropriately determined within the ranges, such as 5 to 50 mm in the length a of the crushed piece instantaneous temperature increase body in the lateral direction, 10 to 100 mm in the length b in the longitudinal direction thereof, and 2 to 10 mm in the height from the bottom of the chamber. Moreover, the values can be appropriately determined within the ranges, such as 20 to 80 mm in the interval c of the crushed piece instantaneous temperature increase body in the lateral direction, 0.5 to 10 mm in the inner diameter of the reactant supply hole, 10 to 50 mm in the distance d from the most downstream end of the crushed piece instantaneous temperature increase body in the conveyance direction to the reactant supply hole in the nearest neighbor column thereof, 20 to 100 mm in the distance e from the most downstream end thereof to the reactant supply holes in the next neighbor column thereof, and 30 to 150 mm in the distance f from the most downstream end thereof to the reactant supply holes in the remotest column thereof.

The reactant such as the oxidation agent and the neutralizing agent to be supplied can be considered in various manners in corresponding to contents or the like of the waste materials to be treated, and for example, gases such as ozone, ammonia, sodium hydroxide and urea are preferable. Ozone functions as the oxidation agent, and ammonia and sodium hydroxide function as the neutralizing agent. If urea is supplied to the place in the neighborhood of the crushed piece instantaneous temperature increase body, the urea is heated by an ambient temperature to a level over 160°C, the urea is decomposed into cyanic acid and ammonia. Cyanic acid is the oxidation agent and ammonia is the neutralizing agent, and therefore both the oxidation agent and the neutralizing agent are supplied by supplying the urea.

The reactant to be supplied by the reactant supply unit is not limited to one kind, and a plurality of kinds of the reactants may be combined and supplied. Moreover, when the unit has a plurality of reactant supply holes as shown in the figure, the reactants to be supplied by the reactant supply holes may be configured to be different from each other.

A temperature of the reactant to be supplied is preferably lower than the softening temperature of the plastics formed into the crushed pieces, and is preferably adjusted to ordinary temperature to 100°C, for example. Instantaneously temperature-increased crushed pieces are immediately cooled by supplying the reactant having such a temperature, a period of time during which softening or decomposition may be caused is shortened, and generation of an unwanted substance such as chlorine can be suppressed.

The non-plastic refuse contained in the crushed pieces is heated by the crushed piece instantaneous temperature increase body. Thus, various gases such as hydrogen sulfide, carbon monoxide, hydrogen and hydrocarbons are produced. Meanwhile, urea supplied to the place in the neighborhood of the crushed piece instantaneous temperature increase body is heated to 160°C or higher to be instantaneously decomposed into an ammonia ion and a cyanate ion, and also a strong oxidation agent such as activated ozone is supplied therein as the reactant. Various gases generated by heating the crushed pieces by the crushed piece instantaneous temperature increase body by supply of the above reactants to the place in the neighborhood of the crushed piece instantaneous temperature increase body are brought into contact with the reactants almost simultaneously with the generation thereof. All of the reactant and various gases are in an activated state. Therefore, an oxidation reaction or a reduction reaction immediately progresses by contact with each other to form a stable salt to leave no room for generating dioxins or the like.

To take an example of such a reaction, when urea is supplied as the reactant, and chlorine is produced from the crushed pieces, the urea is first decomposed into ammonia and cyanic acid, which in turn reacts with an acidic substance such as chlorine to form a salt such as ammonium chloride. Moreover, the cyanic acid and the ammonia produced by instantaneous decomposition also have a role of a catalyst in an ionic state together with the strong oxidation agent such as ozone in the activated state. Moreover, a trace amount of hydrocarbon in the active state or the like produced by heating of the crushed pieces having the non-plastic refuse attached thereon reacts with ozone or the like supplied as the oxidation agent to form formaldehyde, acetaldehyde or the like. Moreover, the strong oxidation agent such as ozone reacts with carbon monoxide to form carbon dioxide, or reacts with hydrogen to form water. Moreover, germicidal action or deodorization action can also be expected by supplying ozone. Moreover, in a special case, hydroxide such as sodium hydroxide having strong alkalinity may be supplied therein as the reactant. Moreover, an acidic substance such as hydrogen chloride is produced by temperature increase of the crushed pieces in several cases, and the acidic substance produced can be neutralized by supplying alkaline hydroxide. When compound molecules produced by multi-dimensional ionic reactions as described above remain in an air flow, the compound molecules are formed into oxide and chloride by a low-temperature pH adjustment dew condensation bed apparatus provided on a downstream of the apparatus, and the oxide and the chloride are collected.

Moreover, the reactant supply holes are arranged in the neighborhood of the crushed piece instantaneous temperature increase body, which configuration is made in order to immediately deliver the reactant to the place in the neighborhood of a decomposed gas produced from the crushed pieces which are brought into contact with the crushed piece instantaneous temperature increase body. The decomposed gas produced from the crushed pieces can be rendered harmless by the multi-dimensional ionic reaction described above.

Moreover, a reaction of the reactant with the gases produced by temperature increase can be further promoted by providing the crushed piece instantaneous temperature increase body and the reactant supply holes in the bottom portion of the chamber. In the bottom portion of the chamber, a state is formed in which the crushed pieces are stacked on the crushed pieces which may be brought into contact with the crushed piece instantaneous temperature increase body. More specifically, a slightly upper portion from a base of the chamber is formed into a state of being covered with the crushed pieces to be stacked. Meanwhile, the inside of the chamber is formed in a negative pressure state by the exhaust unit, and therefore the gases produced from the crushed pieces in the bottom portion of the chamber and the reactant supplied are provided with flowability toward the outlet to some extent while the flowability is being attenuated by the crushed pieces covering an upper portion. The gases and the reactant each having such flowability are to be brought into contact with each other at a high frequency during flowing through a flow path narrowed by stacking of the crushed pieces. Accordingly, the reaction of the reactant with the gases produced from the crushed pieces occurs at a high frequency and with precedence, and therefore no room for progress to the reaction from the gases produced from the crushed pieces to dioxins is left, and as a result, no dioxins are formed.

Moreover, the reactant supply unit may be equipped with a means for preventing clogging of the reactant supply holes. For example, a compressed gas is jetted, in a forward direction relative to a direction of supplying the reactant, into a pipe arranged for supplying the reactant from the reactant supply hole into the chamber. Thus, the crushed pieces or the like stacked in the reactant supply holes can be discharged and removed. The compressed gas may be obtained by compressing the reactant gas, or compressing other gases. Moreover, the unit may be configured in such a manner that a flowmeter is provided in the pipe for supplying the reactant to detect clogging by observing a flow rate of the reactant, and clogging is dissolved by jetting the compressed gas, or the like.

An "air mixture gas supply unit" (0206) causes, to the chamber, supply of an air mixture gas capable of cooling the crushed pieces instantaneously when the crushed pieces instantaneously heated by coming close to the crushed piece instantaneous temperature increase body are isolated therefrom. In Figure 2, the air mixture gas supply unit is provided on a ceiling of the chamber.

The air mixture gas to be supplied by the air mixture gas supply unit is a gas formed by mixing air with a gas other than air, and for example, preferably a gas in which a concentration containing nitrogen is increased in comparison with ordinary air in order to keep the inside of the chamber in an inactive atmosphere. Moreover, the gas may contain the reactant to be supplied by the reactant supply unit. Moreover, the air mixture gas supply unit is also preferably configured so as to have an ozone mixing means for mixing ozone in air to cause supply of an air mixture gas mixed with ozone. Moreover, the present apparatus also causes drying of the organic waste materials inside the chamber to reduce a volume thereof by evaporation of contained moisture, and therefore the air mixture gas is preferably a dried gas.

Moreover, the air mixture gas also fulfills a function of cooling the crushed pieces instantaneously when temperature-increased crushed pieces are isolated from the crushed piece instantaneous temperature increase body, and therefore preferably has a temperature lower than the softening temperature of the plastics formed into the crushed pieces, and the temperature is preferably adjusted to ordinary temperature to 100°C, for example. The air mixture gas having such a temperature is filled inside the chamber. Thus, even when the crushed pieces cause softening or decomposition thereof at the temperature over the softening temperature thereof by coming close to the crushed piece instantaneous temperature increase body, the crushed pieces are cooled instantaneously when the crushed pieces are isolated therefrom. Therefore, the softening or the decomposition can be immediately terminated, and generation of the unwanted substance such as chlorine can be suppressed. Moreover, the air mixture gas having ordinary temperature or lower may be supplied depending on a case. A temperature or a component and a concentration of the gases inside the chamber are configured to be monitorable, and also when an unintended event such as excessive heat generation or combustion is caused, the air mixture gas having ordinary temperature or lower is supplied therein. Thus, such an event can be immediately terminated.

The air mixture gas supply unit may be provided in a lower portion of the chamber in place of being provided in the upper portion of the chamber such as a ceiling, and also may be provided both in the upper portion and the lower portion of the chamber. The inside of the chamber is kept in the negative pressure state, and therefore if the air mixture gas is supplied from the lower portion of the chamber, the air mixture gas is provided with the flowability in a manner similar to the flowability described on the reactant, and evenly spreads inside the chamber.

Moreover, the air mixture gas may be configured to be supplied to the place in the neighborhood of the crushed piece instantaneous temperature increase body. For example, as shown in Figure 5, the holes are provided in the bottom portion of the chamber, and the air mixture gas may be supplied into the chamber. Moreover, the air mixture gas supply unit may be configured in such a manner that the holes provided in the chamber can be used for both supply of the reactant and supply of the air mixture gas. If such a configuration is made, the air mixture gas can be immediately brought into contact with the crushed pieces isolated from the crushed piece instantaneous temperature increase body, and the temperature-increased crushed pieces can be immediately cooled.

Then, a "collection unit" (0209) for collecting the treated organic waste materials is provided under the chamber. The non-plastic refuse contained in the waste materials is reduced in the volume and rendered harmless by dewatering, tissue embrittlement or the like by passing through the treatment which has been described so far, and the plastics having the non-plastic refuse attached thereon are cleaned and rendered harmless. Accordingly, reduction of the volume and rendering the waste materials harmless as a whole can be achieved, and the plastics can be collected in a state to be easily recycled.

In addition, in Figure 2, a configuration is formed in which the chamber is equipped with one screw conveyer, but a configuration may be formed in which the chamber has the screw conveyers arranged in two stages of an upper stage and a lower stage to convey the organic waste materials . Moreover, a configuration is formed in which two or more screw conveyers may be arranged in parallel in the chamber. Moreover, treatment by reciprocating conveyance may be applied. More specifically, treatment while the crushed pieces supplied are conveyed to the other end of the screw conveyer, and then rotation of the screw is reversed to convey the crushed pieces in a direction opposite thereto may be repeatedly applied.

The present apparatus can be configured in corresponding to both continuous treatment and batch treatment of the organic waste materials, and when the batch treatment is applied, a configuration may be formed in which, in a region in which the crushed pieces have already passed in the screw conveyer, energy consumption required for the treatment may be suppressed by suspending operation of the heater for heating the crushed piece instantaneous temperature increase body or supply of the reactant.

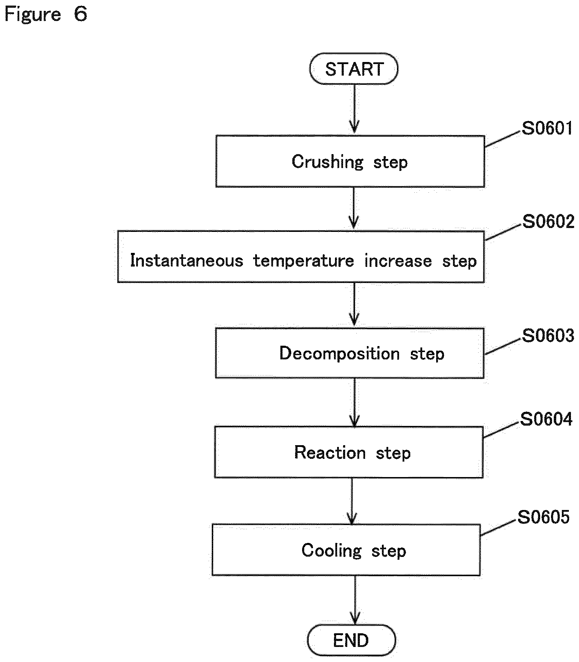

One example of a treatment process of the waste material treatment apparatus according to the present embodiment is shown in a flowchart in Figure 6. As shown in the figure, in the treatment process of the organic waste materials, the organic waste materials containing the plastics are crushed into small pieces (S0601, a crushing step). Then, the temperature of the small pieces crushed in the crushing step is instantaneously increased to a temperature of about a softening temperature thereof (S0602, an instantaneous temperature increase step). The crushed pieces have a meaning same with the meaning of the crushed pieces in the waste material treatment apparatus described above. Moreover, the temperature of the crushed pieces is instantaneously increased by the crushed piece instantaneous temperature increase body. Action by instantaneous temperature increase of the small pieces is similar to the action by temperature increase of the crushed pieces by the crushed piece instantaneous temperature increase body in the waste material treatment apparatus.

Then, instantaneously when the temperature is instantaneously increased in the instantaneous temperature increase step, urea is released to the place near the small pieces, and the urea is decomposed into an ammonia gas and an intermediate of cyanic acid at a temperature near the small piece (S0603, a decomposition step). As described above, the urea released to the place near temperature-increased small pieces is decomposed into the ammonia gas and the cyanic acid generally at 160°C or higher. As a means for releasing the urea to the place near the temperature-increased small pieces, the reactant supply unit in the waste material treatment apparatus described above corresponds thereto, for example.

Then, both substances decomposed in the decomposition step are allowed to react with various gases generated from instantaneously temperature-increased small pieces (S0604, a reaction step) . For example, when chlorine is produced from the small pieces, the chlorine reacts with the ammonia and the cyanic acid formed by decomposition, and ammonium chloride is formed. Active chlorine generated from the small pieces is immediately allowed to react therewith into stable ammonium chorine. Thus, the chorine reacts with hydrocarbon contained in the organic waste materials, hydrocarbon produced from the organic waste materials or the like, and the waste materials are rendered harmless.

Then, the substances produced by the reaction therewith in the reaction step are cooled (S0605, a cooling step) . For example, when ammonium chloride is formed in the reaction step, a further reaction can be suppressed by being cooled. Moreover, the cooling step is effective in cooling the temperature-increased small pieces near the place, and also softening or decomposition of the small pieces as caused by the temperature increase can be suppressed. In addition, in the reaction step and the cooling step, the reaction step is not necessarily performed before the cooling step, and the reaction step and the cooling step may be performed in reverse order, or both steps may be performed in parallel.

Moreover, the reaction step may further have a hydroxide reaction substep of releasing hydroxide to the place near the small pieces to allow reaction therewith. An acidic substance such as hydrogen chloride is formed by increasing the temperature of the small pieces in several cases. Then, the formed acidic substance can be neutralized by releasing hydroxide such as sodium hydroxide to the place near the small pieces.

Moreover, a treatment process performed in the waste material treatment apparatus according to the present embodiment can also be described as a method for treating crushed pieces being waste materials as described below. More specifically, the treatment process can be described as a method for treating crushed pieces being waste materials, including: an instantaneous temperature increase step of instantaneously increasing a temperature in the neighborhood of a surface of the crushed piece to a temperature in the neighborhood of a softening temperature thereof; a temperature decrease step of instantaneously decreasing a temperature in the neighborhood of the surface of the instantaneously temperature-increased crushed pieces; and a reactant gas supply step of supplying a reactant gas being an oxidation gas and a neutralizing gas to a place in the neighborhood of the surface of the instantaneously temperature-increased crushed pieces. The instantaneous temperature increase step is mainly performed by a configuration such as the crushed piece instantaneous temperature increase body. Therefore, the temperature decrease step is mainly performed by a configuration such as the air mixture gas supply unit. Then, the reactant gas supply step is mainly performed by a configuration such as the reactant supply unit.

Moreover, the treatment process can be described as a method for treating crushed pieces being waste materials, including: an instantaneous temperature increase step of instantaneously increasing a temperature in the neighborhood of a surface of the crushed pieces to a temperature in the neighborhood of a softening temperature thereof; and a reactant gas supply step of supplying a reactant gas being an oxidation gas and a neutralizing gas each having a temperature relatively lower than the temperature in the neighborhood of the softening temperature thereof to a place in the neighborhood of the surface of the crushed pieces to instantaneously decrease a temperature in the neighborhood of the surface of the instantaneously temperature-increased crushed pieces. The instantaneous temperature increase step is mainly performed by a configuration such as the crushed piece instantaneous temperature increase body, and the reactant gas supply step is mainly performed by a configuration such as the reactant supply unit.

Moreover, the treatment process can be described as a method for treating crushed pieces being waste materials, including: an instantaneous temperature increase step of instantaneously increasing a temperature in the neighborhood of a surface of the crushed pieces to a temperature in the neighborhood of a softening temperature thereof; a reactant gas supply step of supplying a reactant gas being an oxidation gas and a neutralizing gas each having a temperature relatively lower than the temperature in the neighborhood of the softening temperature thereof to a place in the neighborhood of the surface of the crushed pieces to instantaneously decrease a temperature in the neighborhood of the surface of instantaneously temperature-increased crushed pieces; and a temperature decrease step of instantaneously decreasing the temperature in the neighborhood of the surface of the instantaneously temperature-increased crushed pieces. The instantaneous temperature increase step is mainly performed by a configuration such as the crushed piece instantaneous temperature increase body, and the reactant gas supply step is mainly performed by a configuration such as the reactant supply unit. Then, the temperature decrease step is mainly performed by a configuration such as the air mixture gas supply unit.

Moreover, the treatment process can be described as a method for treating crushed pieces being waste materials, including: an instantaneous temperature increase step of instantaneously increasing a temperature in the neighborhood of a surface of the crushed pieces to a temperature in the neighborhood of a softening temperature thereof; and a reactant gas supply step of supplying a reactant gas being an oxidation gas and a neutralizing gas each having a temperature relatively lower than the temperature in the neighborhood of the softening temperature thereof to a place in the neighborhood of the surface of the crushed pieces to allow an active gas generated from the place in the neighborhood of the surface of the crushed piece to react therewith and instantaneously decrease a temperature in the neighborhood of the surface of the instantaneously temperature-increased crushed pieces. The instantaneous temperature increase step is mainly performed by a configuration such as the crushed piece instantaneous temperature increase body, and the reactant gas supply step is mainly performed by a configuration such as the reactant supply unit.

### <Other configurations>

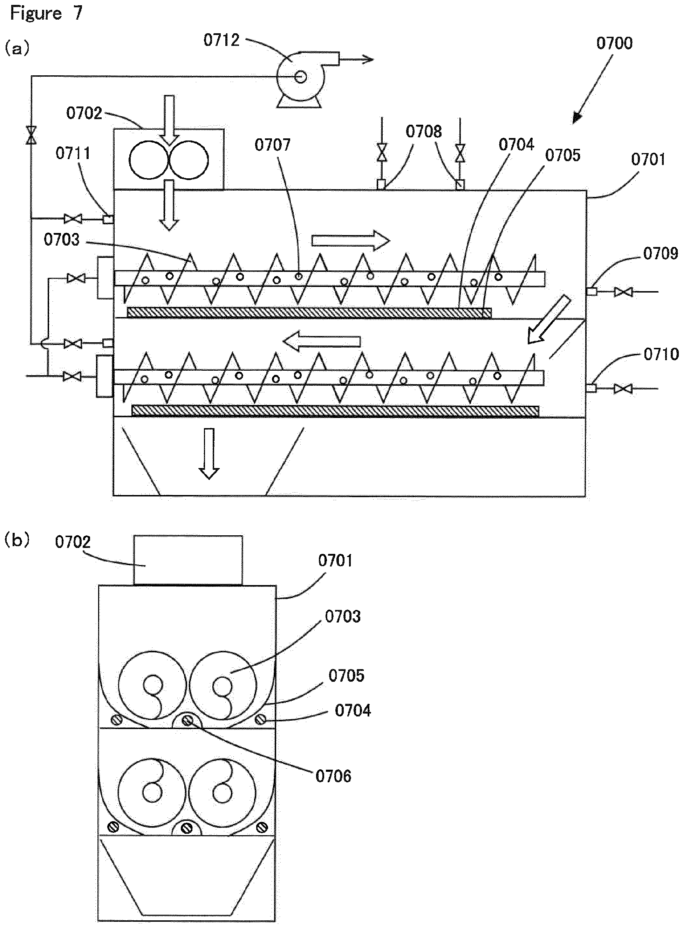

Figure 7 (a) is a conceptual diagram showing one example of a waste material treatment apparatus in another aspect in Embodiment 1. Moreover, Figure 7(b) is a cross-sectional view of the present apparatus shown in Figure (a) when viewed from a conveyance direction of the crushed pieces. In addition, in Figure 7(b), a pipe for supplying the reactant or the air mixture gas is not shown.

A "waste material treatment apparatus" (0700) has a "chamber" (0701) for applying heating treatment to the waste materials. In the present aspect, the chamber having a "screw conveyer" (0703) is configured in two stages of an upper stage and a lower stage. A standardized product has a configuration in one stage in several cases. A "crushed piece supply port" (0702) is similar to the crushed piece supply port in the previous aspect shown in Figure 2 or the like, and therefore the description is omitted.

A "crushed piece instantaneous temperature increase body" is heated by a heater for repeatedly performing an instantaneous temperature increase of the crushed pieces supplied into the chamber. In Figure 7, a "chamber bottom portion" (0705) on which a "heater" (0704) is arranged corresponds to the crushed piece instantaneous temperature increase body. As shown in Figure 7(a) and Figure 7(b), a rod-shaped "heater" (0704) is installed along the conveyance direction in a place in the neighborhood of an outer side of the bottom portion. Moreover, a rod-shaped "heater" (0706) is also installed between two screws in the bottom portion of the chamber.

A place in the neighborhood of the heater on a bottom surface of the chamber is heated by the heater, and when the crushed pieces to be conveyed inside the chamber are brought into contact with the place (hereinafter, also referred to as a heater-neighborhood place), the crushed pieces are heated. The heater-neighborhood place is a part of the chamber bottom surface, and the crushed pieces are conveyed while being agitated, and therefore the crushed pieces are not always brought into contact with the heater-neighborhood place, and are conveyed while being occasionally brought into contact therewith and being occasionally separated therefrom. The instantaneous temperature increase of the crushed pieces can be repeatedly performed by conveying the crushed pieces as described above. In order to repeat such instantaneous temperature increase, the apparatus is preferably equipped with a mechanism for agitating the crushed pieces, such as the screw conveyer. As a conveying means other than the screw conveyer, a vibration conveyer for conveying an object while vibrating the object may be used.

In addition, installation of the heater is not limited to the aspect described above, and for example, the rod-shaped heaters along the bottom surface of the chamber may be installed in arrangement, such as sleepers, at an interval in a direction substantially perpendicular to the conveyance direction. Moreover, the heater only needs be a device which can emit heat, and fuel also does not matter.

Moreover, a pipe may be arranged in the bottom portion of the chamber, and oil heated by the heater may be introduced into the pipe. A heater control unit for controlling the heater is similar to the heater control unit described in the previous aspect or the like, and therefore the description is omitted.

The reactant supply unit includes the reactant supply hole arranged in the neighborhood of the crushed piece instantaneous temperature increase body to allow gases produced by temperature increase to react therewith in the neighborhood of the crushed piece instantaneous temperature increase body. In Figure 7, a plurality of "reactant supply holes" (0707) are provided in a shaft of a screw of the screw conveyer. Moreover, the reactant supply holes may be provided in the shaft of the screw, and also may be configured so as to be arranged in the neighborhood of the heater provided in the chamber bottom portion, for example. The reactant to be supplied, the aspect of supply and the action by the reactant to be supplied have been already described, and therefore the description is omitted.

An "air mixture gas supply unit" (0708) causes, to the chamber, supply of an air mixture gas capable of cooling, instantaneously when the instantaneously temperature-increased crushed pieces by coming close to the crushed piece instantaneous temperature increase body are isolated from the crushed piece instantaneous temperature increase body, the crushed pieces isolated therefrom. In Figure 7, the air mixture gas supply units are not only provided on a ceiling of the chamber, but also provided in a sidewall part of the chamber in each stage (0709, 0710). The air mixture gas supply unit is also basically similar to the air mixture gas supply unit shown in the previous aspect or the like, and therefore the description is omitted.

Moreover, an exhaust unit configured by a "negative pressure outlet" (0711), a "pump" (0712) and the like, and ability of collecting the treated organic waste materials are also similar to the previous aspect, and therefore the description is omitted.

As described above, the waste material treatment apparatus and the waste material treatment method according to the present embodiment are innovative, in which the waste materials are rendered harmless and a volume thereof is reduced, and also generation of dioxins is eliminated, and also a combustion aid of oil and a natural gas is omitted by no application of high-temperature combustion and high-temperature oxidation incineration, and metals in the waste materials can be obviously recycled, and plastics therein can also be recycled by low-temperature treatment for rendering the waste materials harmless. Moreover, a solar generator is used as an energy source for operating the apparatus. Thus, generation of carbon dioxide causing global warming can be suppressed, and reduced, which can contribute to inhibition of global warming.

### <Embodiment 1 effect>

The waste material treatment apparatus and the waste material treatment method can be provided, in which the waste materials are rendered harmless and the volume thereof is reduced, and also the plastics can be recycled without discharging hazardous substances such as dioxins.

### <Embodiment 2>

### <Embodiment 2 outline>

A waste material treatment apparatus according to the present embodiment has features of applying Embodiment 1 as a base, and having a means for analyzing gases in a chamber to control a heater, or the like in corresponding to the analysis results.

### <Embodiment 2 configuration>

The waste material treatment apparatus according to the present embodiment has an intra-chamber gas analysis unit, in which a heater control unit has an intra-chamber gas-dependent control means for controlling the heater in corresponding to the analysis results of the intra-chamber gases in the intra-chamber gas analysis unit.

In the intra-chamber gas analysis unit, specifically, a known gas concentration sensor or the like is provided in corresponding to various gases to be analyzed. Specific examples of the gases to be analyzed mainly include a chlorine gas, a hydrogen gas and a carbon monoxide gas. As described in Embodiment 1, the chlorine gas is a substance causing generation of hazardous substances such as dioxins. Moreover, the hydrogen gas is a substance being highly combustible and explosive, and therefore is the gas to be monitored. The carbon monoxide is a substance serving as a base of carbon dioxide causing global warming, and therefore is the gas to be monitored. Moreover, dioxins or carbon dioxide per se may be applied as an object to be analyzed.

Specific aspects in which the intra-chamber gas analysis unit is provided in the waste material treatment apparatus can be considered in various manners, and are not specified in one aspect. For example, a detection means for detecting the gases to be analyzed, such as a sensor, may be provided inside the chamber, and a configuration may be formed in such a manner that a gas discharged to an outside of the chamber through a negative pressure outlet is introduced to the detection means.

In the intra-chamber gas-dependent control means, for example, when a concentration of a chlorine gas is higher than an intended concentration in the analysis results, heating in the heater is suppressed to suppress a degree of temperature increase by the crushed piece instantaneous temperature increase body for the crushed pieces. Such operation is similar also in the case where hydrogen or carbon monoxide is applied as the gas to be analyzed.

Moreover, the intra-chamber gas analysis unit and the intra-chamber gas-dependent control means may be configured in such a manner that an aspect of heater control in corresponding to a kind of gas to be analyzed or the analysis results may be appropriately selected in corresponding to a content of the waste materials to be treated. In the organic waste materials to be treated, various waste materials are mixed, and the gases to be produced are different according to the aspect thereof, or a concentration assumed for each gas or an allowable concentration is different in several cases. Accordingly, the intra-chamber gases are preferably analyzed in corresponding to the waste materials to be treated, and the heater control in corresponding to the results is preferably performed.

Moreover, the waste material treatment apparatus according to the present embodiment may be configured in such a manner that not only heat is controlled, but also supply of the reactant or supply of the air mixture gas is controlled in corresponding to the analysis results of the intra-chamber gases. More specifically, a reactant supply unit may have an intra-chamber gas-dependent neutralizing agent supply control means for controlling supply of the reactant in corresponding to the analysis results of the intra-chamber gases in the intra-chamber gas analysis unit, and an air mixture gas supply unit may have an intra-chamber gas-dependent air mixture gas supply control means for controlling the air mixture gas in corresponding to the analysis results of the intra-chamber gases in the intra-chamber gas analysis unit.

For example, as an aspect of control by the intra-chamber gas-dependent neutralizing agent supply control means, when a concentration of a chlorine gas is over an intended range or an allowable range in the analysis results, an amount of supply of urea for allowing the chlorine gas to react therewith is increased, and so on. Moreover, when a plurality of reactants which can be supplied therein are available, supply of any reactant is preferably controlled, or when the plurality of reactants are supplied in combination therewith, a supply ratio of each reactant is also preferably controlled. Moreover, an aspect of control in the intra-chamber gas-dependent air mixture gas supply control means is similar thereto.

Moreover, a conveying speed in a conveyance means such as a screw conveyer may be controlled in corresponding to the analysis results of the intra-chamber gases. Moreover, when the crushed pieces are conveyed in a reciprocating manner, whether or not reciprocating conveyance is continued may be controlled in corresponding to the analysis results of the intra-chamber gases. Moreover, timing of completing the waste material treatment may be configured to be controlled in corresponding to the analysis results of the intra-chamber gases.

### <Embodiment 2 effect>

The waste materials can be further treated in a safe and intended aspect by controlling each unit by feeding back the analysis results in the intra-chamber gas analysis unit.

### <Embodiment 3>

### <Embodiment 3 outline>

A waste material treatment apparatus according to the present embodiment has features of applying Embodiment 1 or Embodiment 2 as a base, in which a crushed piece instantaneous temperature increase body is a needle body which performs an insertion and retraction movement relative to the crushed pieces inside the chamber.

### <Embodiment 3 configuration>

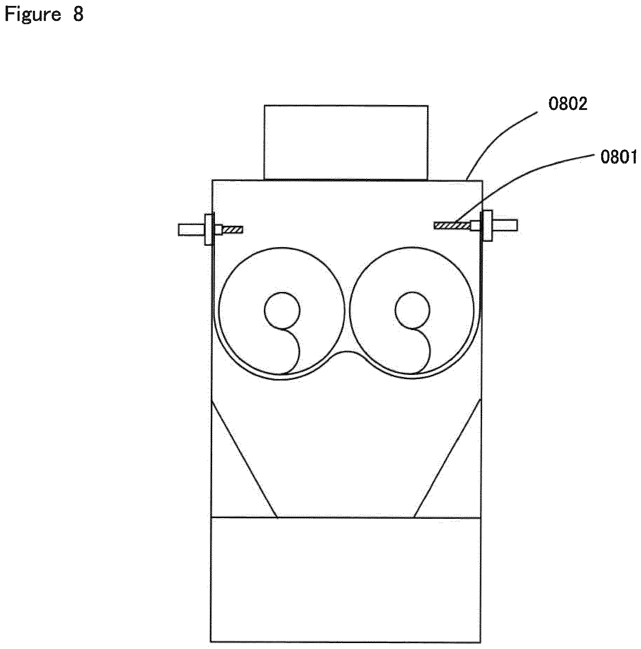

Figure 8 is a conceptual diagram showing one example of a waste material treatment apparatus according to the present embodiment. The present figure shows a cross-sectional view of a screw conveyer when viewed from a conveyance direction thereof. The crushed piece instantaneous temperature increase body in the present embodiment is the needle body which performs the insertion and retraction movement relative to the crushed pieces inside the chamber. As shown in the figure, a "needle body" (0801) is provided on a side surface of a "camber" (0802) so as to perform the insertion and retraction movement toward an inside of the chamber (802). A configuration in which the needle body performs the insertion and retraction movement is not limited, but a configuration may be formed in which elements such as a motor serving as a power source, a cam and a shaft are combined, for example.

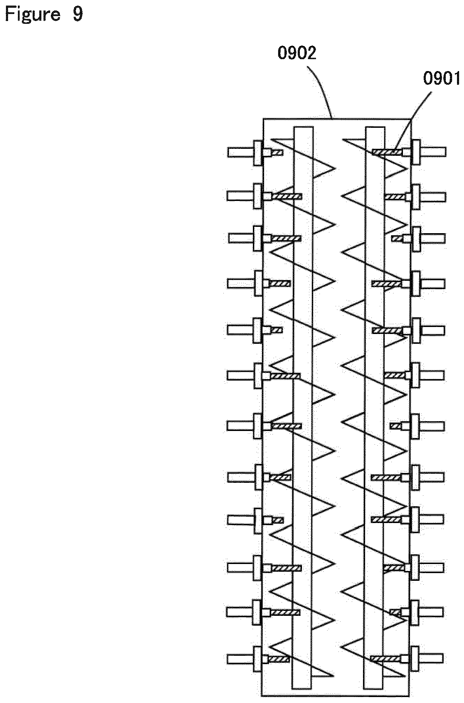

Figure 9 shows a conceptual diagram obtained by viewing the organic waste material treatment apparatus in Figure 8 from above. As shown in the figure, a "needle body" (0901) is configured so as to perform the insertion and retraction movement toward an inside of a "chamber" (0902), and equipped with a great number of needle bodies.

A speed of insertion and retraction only needs be appropriately selected in corresponding to an object to be treated or a treatment amount, and if the insertion and retraction movement is performed at about 20 to about 100 reciprocations per minute, such a case is preferable, for example. Moreover, a configuration may be formed, in which the intra-chamber gas analysis unit described in Embodiment 2 is provided, and also timing of the insertion and retraction movement, a temperature of the needle body, or the like is be adjusted in corresponding to the analysis results.

When the crushed piece instantaneous temperature increase body is configured as described above, the crushed pieces are brought into contact with the needle body in a significantly short period of time by the insertion and retraction movement of the needle body relative to the crushed pieces conveyed while being agitated by the screw. Thus, a temperature of the crushed pieces in contact therewith can be instantaneously increased in an effective manner.

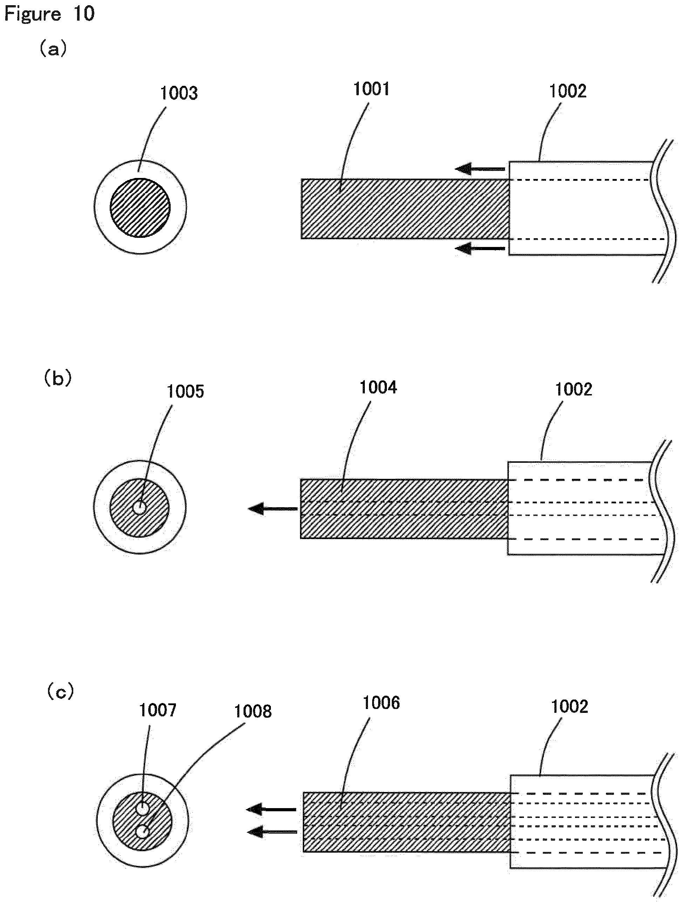

Moreover, the needle body may be provided with a reactant supply hole. Figure 10 is a conceptual diagram showing one example of the needle body provided with the reactant supply hole. In Figure 10 (a), a "needle body" (1001) performs the insertion and retraction movement by moving in and out of a "tubular member" (1002) provided in an inner wall of the chamber. A reactant is supplied from an inside of the tubular member. More specifically, a "gap between the inner wall surface of the tubular member and the needle body" (1003) serves as the reactant supply hole.

Moreover, in Figure 10(b), a "needle body" (1004) is equipped with one "hole" (1005) axially penetrating though the needle body, and the hole serves as the reactant supply hole. As shown in Figure 10(c), "two holes" (1007, 1008) axially penetrating through a "needle body" (1006) may be provided, and may be applied as the reactant supply holes. Thus, when the needle body is equipped with a plurality of holes, a configuration may be formed, in which different reactants are supplied from the holes, respectively, or the reactant is supplied from one hole, and also the air mixture gas is supplied from any other hole.

With regard to supply of the reactant from the reactant supply hole, a configuration may be formed, in which the reactant is supplied all the time while the waste materials are being treated, or is supplied at timing in corresponding to the insertion and retraction movement of the needle body. For example, a configuration may be formed, in which no reactant is supplied while the needle body performs movement out of the tubular member, and the reactant is supplied while the needle body performs a movement of returning to the tubular member. The needle body performs the movement of returning thereto to be separated from the crushed piece which has been in contact with the needle body so far. However, the gases produced by temperature increase can be allowed to immediately react with the reactant by supplying the reactant on the above occasion.

### <Embodiment 3 effect>

According to the present embodiment, the temperature of the crushed pieces can be instantaneously increased, temperature-increased crushed pieces can be cooled, and the gases produced can be immediately neutralized.

### Reference Signs List

0200 Organic waste material treatment apparatus
0201 Chamber
0202 Crushed piece supply port
0203 Screw conveyer
0204 Crushed piece instantaneous temperature increase body
0205 Reactant supply hole
0206 Air mixture gas supply port
0207 Negative pressure outlet
0208 Pump
0209 Collection unit

## Claims

1. A waste material treatment apparatus comprising:
a chamber for heating treatment of waste materials;
a crushed piece supply port for supplying crushed pieces of waste materials to the chamber;
a crushed piece instantaneous temperature increase body heated by a heater for repeatedly performing instantaneous temperature increase of the crushed pieces supplied into the chamber;
a heater control unit capable of controlling the heater, when plastics are contained in the crushed pieces, in such a manner that a temperature is instantaneously increased to a temperature in the neighborhood of a softening temperature of the plastics by the crushed piece instantaneous temperature increase body;
a reactant supply unit including a reactant supply hole for supplying a reactant being an oxidation agent and a neutralizing agent arranged in the neighborhood of the crushed piece instantaneous temperature increase body to allow gases produced from the crushed pieces by temperature increase to react therewith in the neighborhood of the crushed piece instantaneous temperature increase body;
an air mixture gas supply unit for supplying, to the chamber, an air mixture gas capable of cooling, instantaneously when instantaneously temperature-increased crushed pieces by coming close to the crushed piece instantaneous temperature increase body are isolated from the crushed piece instantaneous temperature increase body, the crushed pieces isolated therefrom; and
an exhaust unit having a negative pressure outlet for negatively pressurizing an inside of the chamber to discharge gases inside the chamber to an outside.

2. The waste material treatment apparatus according to claim 1, wherein the gas supply unit has an ozone mixing means for mixing ozone in air.

3. The waste material treatment apparatus according to claim 1 or 2, wherein the negative pressure outlet is provided in an upper portion of the chamber.

4. The waste material treatment apparatus according to any one of claims 1 to 3, further comprising an intra-chamber gas analysis unit,
wherein the heater control unit has an intra-chamber gas-dependent control means for controlling the heater in corresponding to analysis results of intra-chamber gases in the intra-chamber gas analysis unit.

5. The waste material treatment apparatus according to any one of claims 1 to 4, wherein the reactant supply hole is provided for the crushed piece instantaneous temperature increase body.

6. The waste material treatment apparatus according to any one of claims 1 to 5, wherein the crushed piece instantaneous temperature increase body is a needle body which performs an insertion and retraction movement relative to the crushed pieces inside the chamber.

7. The waste material treatment apparatus according to any one of claims 1 to 6, further comprising a crushed piece agitation mechanism for agitating the crushed pieces inside the chamber.

8. The waste material treatment apparatus according to claim 7, wherein the crushed piece instantaneous temperature increase body is a fixed block arranged in such a manner that the crushed pieces agitated inside the chamber come instantaneously close thereto.

9. A method for treating waste materials, comprising:
a crushing step of crushing the waste materials into small pieces;
an instantaneous temperature increase step of instantaneously increasing, when plastics are contained in crushed pieces crushed in the crushing step, a temperature of the plastics to a temperature of about a softening temperature of the plastics;
a decomposition step of releasing urea to a place near temperature-increased crushed pieces instantaneously when the temperature is instantaneously increased in the instantaneous temperature increase step to decompose the urea into an ammonia gas and an intermediate of cyanic acid at a temperature near the crushed pieces;
a reaction step of allowing both substances decomposed in the decomposition step to react with various gases generated from instantaneously temperature-increased crushed pieces; and
a cooling step of cooling the substances produced by the reaction in the reaction step.

10. The method for treating waste materials according to claim 9, wherein the reaction step further has a hydroxide reaction substep of releasing hydroxide to a place near the crushed pieces to allow reaction therewith.

11. A method for treating crushed pieces being waste materials, comprising:
an instantaneous temperature increase step of instantaneously increasing a temperature in the neighborhood of a surface of the crushed piece to a temperature in the neighborhood of a softening point thereof;
a temperature decrease step of instantaneously decreasing the temperature in the neighborhood of the surface of instantaneously temperature-increased crushed pieces; and
a reactant gas supply step of supplying a reactant gas being an oxidation gas and a neutralizing gas to a place in the neighborhood of the surface of instantaneously temperature-increased crushed pieces.

12. A method for treating crushed pieces being waste materials, comprising:
an instantaneous temperature increase step of instantaneously increasing a temperature in the neighborhood of a surface of the crushed pieces to a temperature in the neighborhood of a softening point thereof; and
a reactant gas supply step of supplying an reactant gas being an oxidation gas and a neutralizing gas each having a temperature relatively lower than the temperature in the neighborhood of the softening point thereof to a place in the neighborhood of a surface of instantaneously temperature-increased crushed pieces to instantaneously decrease the temperature in the neighborhood of the surface of the instantaneously temperature-increased crushed pieces.

13. A method for treating crushed pieces being waste materials, comprising:
an instantaneous temperature increase step of instantaneously increasing a temperature in the neighborhood of a surface of the crushed piece to a temperature in the neighborhood of a softening point thereof;
a reactant gas supply step of supplying a reactant gas being an oxidation gas and a neutralizing gas to a place in the neighborhood of a surface of instantaneously temperature-increased crushed pieces to allow an active gas generated from the place in the neighborhood of the surface of the crushed piece to react therewith; and
a temperature decrease step of instantaneously decreasing the temperature in the neighborhood of the surface of the instantaneously temperature-increased crushed pieces.

14. A method for treating crushed pieces being waste materials, comprising:
an instantaneous temperature increase step of instantaneously increasing a temperature in the neighborhood of a surface of the crushed pieces to a temperature in the neighborhood of a softening point thereof; and
a reactant gas supply step of supplying a reactant gas being an oxidation gas and a neutralizing gas each having a temperature relatively lower than the temperature in the neighborhood of the softening point thereof to a place in the neighborhood of a surface of instantaneously temperature-increased crushed pieces to allow an active gas generated from the place in the neighborhood of the surface of the crushed pieces to react therewith, and also to instantaneously decrease the temperature in the neighborhood of the surface of the instantaneously temperature-increased crushed pieces.
